(19) Europäisches Patentamt
European Patent Office
Office européen des brevets

(11) Veröffentlichungsnummer: **0 039 471**
**A1**

(12) # EUROPÄISCHE PATENTANMELDUNG

(21) Anmeldenummer: **81103200.2**

(22) Anmeldetag: **29.04.81**

(51) Int. Cl.³: **C 07 C 19/08**, C 07 C 17/10

(30) Priorität: **05.05.80 DE 3017154**

(43) Veröffentlichungstag der Anmeldung: **11.11.81**
**Patentblatt 81/45**

(84) Benannte Vertragsstaaten: **BE DE FR GB IT NL**

(71) Anmelder: **HOECHST AKTIENGESELLSCHAFT,**
**Postfach 80 03 20, D-6230 Frankfurt/Main 80 (DE)**

(72) Erfinder: **von Halasz, Sigmar-Peter, Dr.,**
**Feldbergstrasse 50, D-6233 Kelkheim (Taunus) (DE)**

(54) **Verfahren zur Herstellung von 2-Chlor-1,1,1,2,3,3,3-heptafluorpropan.**

(57) Die Erfindung betrifft ein Verfahren zur selektiven Herstellung von 2-Chlor-1,1,1,2,3,3,3-heptafluorpropan. Dabei wird 1,1,1,2,3,3,3-Heptafluorpropan in der Gasphase mit elementarem Chlor in Gegenwart von energiereicher Strahlung bei Reaktionstemperaturen im Bereich von –30 bis +500 °C umgesetzt.

EP 0 039 471 A1

0039471

HOECHST AKTIENGESELLSCHAFT HOE 80/F 085 Dr.SP/cr

# Verfahren zur Herstellung von 2-Chlor-1,1,1,2,3,3,3-heptafluorpropan

Die vorliegende Erfindung betrifft ein Verfahren zur selektiven Herstellung von 2-Chlor-1,1,1,2,3,3,3-heptafluor-propan ("2-Chlor-heptafluorpropan") durch Photochlorierung von 1,1,1,2,3,3,3-Heptafluorpropan ("2H-Heptafluorpropan").

2 -Chlor-heptafluorpropan (Siedepunkt -2°C) gehört zur Substanzklasse der vollständig halogenierten Alkane, die sich durch eine besondere thermische und chemische Stabilität, durch Unbrennbarkeit sowie durch besondere elektrische und andere physikalische Eigenschaften auszeichnet. 2-Chlor-heptafluorpropan eignet sich unter anderem als Kühlmittel oder Wärmeüberträger, als schonendes Lösemittel oder Reinigungsmittel für tiefe Temperaturen, als gasförmiges oder flüssiges Inertmedium, beispielsweise als Dielektrikum oder Isoliermedium, als Feuerlöschmittel oder Zusatzkomponente für Feuerlöschmittel oder als Ätzungsmittel für Siliziumdioxid-Schichten auf Silizium. In der Patentliteratur wurde 2-Chlor-heptafluorpropan als Treibmittel oder Treibmittelkomponente (US-Patent 40 57 973) und für die Herstellung von löslichen, schmelzbaren und schwer entflammbaren Polymeren aus leichter entflammbaren Polymeren durch Bestrahlung (DE-PS 12 13 117) vorgeschlagen. An einem einfachen, wirtschaftlichen und umweltfreundlichen Verfahren zur Herstellung der Titelverbindung besteht daher erhebliches Interesse.

2-Chlor-heptafluorpropan ist bereits mehrfach in der Literatur beschrieben worden. Die meisten Verfahren zu seiner Herstellung beruhen auf der Fluorierung einer

$C_3$-Verbindung:

So kann gemäß US-Patent 24 66 189 durch Fluorierung von 2-Chlor-pentafluorpropen mit Fluorwasserstoff und Bleidioxid im Autoklaven oder gemäß britischer Patentschrift 839 034 durch Addition von elementarem Fluor an 2-Chlor-pentafluorpropen die Verbindung $CF_3CClF\ CF_3$ erhalten werden. Auch bei der Fluorierung von 1,1,1-Trifluor-trichlorpropen mittels Kobalttrifluorid gemäß US-Patent 26 70 387 fällt 2-Chlor-heptafluorpropan mit niedriger Ausbeute an.

Bei der Fluorierung von 3-Chlor-propen- 2 mit Fluorwasserstoff bei Temperaturen von über 500°C entsteht nach dem Verfahren der US-Patentschrift 30 47 641 in einer Ausbeute von etwa 50 % die Titelverbindung. Bei der Umsetzung von Fluor oder Chlortrifluorid mit $CF_3CCl_2CClF_2$ oder $CF_3CCl_2CCl_2F$ in Gegenwart eines Aluminiumfluoridkatalysators bildet sich gemäß US-Patent 28 31 035 neben Octafluorpropan auch 2-Chlor-heptafluorpropan.

Im Labormaßstab kann man die Titelverbindung durch Addition von Chlorfluorid an Hexafluorpropen in Ausbeuten von über 80 % herstellen (D.D. Moldavski, V.G. Temchenko et al., Zh. Org. Khim. 9 , 673). Eine Übertragung dieses Verfahrens in den technischen Maßstab ist jedoch schwierig, da schwer abtrennbare Nebenprodukte anfallen und das eingesetzte Chlorfluorid hoch-toxisch ist, unbequem zu handhaben ist, da es z.B. mit Glas heftig und mit wasserstoffhaltigen Verbindungen explosionsartig reagiert und überdies seinerseits aus elementarem Fluor und Chlor erst bereitet werden muß.

Keines der bekannten Verfahren eignet sich zur selektiven Herstellung von 2-Chlor-heptafluorpropan in hohen Ausbeuten. Bei allen Verfahren kommt es in beträchtlichem Ausmaß zu Fragmentierungen oder anderen Konkurrenzreaktionen. Die unerwünschten Nebenprodukte verringern die Ausbeute und machen umfangreiche Destillationen erforderlich. Darüberhinaus erfordern die bekannten Herstellmethoden schwer zugängliche Ausgangsverbindungen, unwirtschaftliche Fluorierungsmittel oder hohe Reaktionstemperaturen, die erhöhte Korrosion der Reaktormaterialien und kurze Standzeiten von Katalysatoren zur Folge haben.

Es bestand daher die Aufgabe, auf der Basis leicht zugänglicher Ausgangsmaterialien ein einfaches Verfahren zur selektiven Herstellung von 2-Chlor-heptafluorpropan zu finden, das mit hohen Ausbeuten verläuft.

Die im Hauptanspruch definierte Erfindung löst dieses Problem.

Das erfindungsgemäße Verfahren kann kontinuierlich nach Art bekannter Gas/ Gas-Reaktionen ausgestaltet werden. Ein Feststoffkatalysator ist dabei nicht erforderlich. Bevorzugt ist ein Temperaturbereich von 20 bis 450°C, insbesondere 30 bis 400°C, vorzugsweise 40 bis 350°C.

Unter energiereichem Licht wird hier Strahlung verstanden, die in der Lage ist, Chlormoleküle in Chloratome zu zerlegen, insbesondere sichtbares Licht und ultraviolettes Licht im Bereich von 450 bis 260 nm. Das erfindungsgemäße Verfahren läßt sich durch folgende Gleichung $CF_3\text{-}CHF\text{-}CF_3 + Cl_2 \xrightarrow{h\nu} CF_3\text{-}CClF\text{-}CF_3 + HCl$ beschreiben.

Die Reaktion läßt sich in einer üblichen Bestrahlungsapparatur, wie sie z.B. zur Chlorierung von Toluol zu Benzylchlorid verwendet wird, durchführen. Eine solche Apparatur besteht z.B. aus einem Glaskolben, in den eine mit einem Quarzrohr versehene Quecksilber-Hochdruck-Tauchlampe eingeführt wird. Weiterhin ist der Glaskolben mit Gaseinleitungsrohr, Innenthermometer,

einem Kondensor, der mit Kohlendioxid gekühlt wird und einem am Boden angebrachten Ablaßhahn ausgestattet. Der Glaskolben soll in Höhe der Tauchlampe und in Höhe des Ablaßhahns von außen beheizt werden können.

In den Glaskolben werden die dosierten gasförmigen Ausgangsprodukte im allgemeinen als Gemisch eingeleitet. Das den Reaktor verlassende Gasgemisch wird mit Wasser gewaschen, wobei entstandener Chlorwasserstoff absorbiert wird. Anschließend wird mit Thiosulfatlösung und verdünnter Natronlauge das restliche Chlor entfernt und mit Türmen, die mit Kalziumchlorid bzw. mit Phosphorpentoxid gefüllt sind, getrocknet. Das Rohprodukt läßt sich in geeigneten Kühlfallen kondensieren.

Die Siedepunkte von Ausgangsprodukten und Endprodukten sind in der folgenden Tabelle zusammengefaßt:

| Produkt | Formel | Molgew. | Kp./1 bar |
|---|---|---|---|
| 2H-Heptafluorpropan | $CF_3CHFCF_3$ | 170,03 | -18°C |
| Chlor | $Cl_2$ | 70,96 | -34,1° |
| Chlorwasserstoff | HCl | 36,46 | -85° |
| 2Cl-Heptafluorpropan | $CF_3CClFCF_3$ | 204,47 | -2° |

Das eingesetzte 2H-Heptafluorpropan wird in technischer Reinheit, zweckmäßigerweise in wasserfreier Form eingesetzt, d.h. vorzugsweise mit Phosphorpentoxid getrocknet. 2H-Heptafluorpropan ist in einfacher Weise durch die quantitativ verlaufende Addition von Fluorwasserstoff an Hexafluorpropen, z.B. gemäß DE-OS 27 12 732 verfügbar. Chlor wird einer handelsüblichen Stahlflasche entnommen und zweckmäßigerweise in wasserfreier Form eingesetzt, d.h. beispielsweise mit konzentrierter Schwefelsäure getrocknet.

Der Umsatz an 2H-Heptafluorpropan beträgt bei 300° und

Atmosphärendruck, je nach der Strahlungsintensität, etwa 0,1 bis 3 Mol/l Reaktorvolumen und Stunde. Niedrigere Durchsätze sind ohne weiteres möglich. Bei höheren Drucken kann der Umsatz entsprechend höher liegen. Die Zugabe von Chlor erfolgt im allgemeinen ohne Verdünnung; die Menge an Chlor liegt dabei im allgemeinen zwischen 0,1 und 3,5 Mol/l Reaktorvolumen und Stunde. Die Chlormenge soll der Menge des gleichzeitig und kontinuierlich zugegebenen 2H-Heptafluorpropans mindestens äquivalent sein. Bevorzugt ist ein geringer Chlorüberschuß. Die Molverhältnisse 2H-Heptafluorpropan/Chlor liegen beispielsweise zwischen 1 : 1,5 und 1:1, vorzugsweise zwischen 1 : 1,15 und 1 : 1,05. Durch einen Überschuß an Chlor läßt sich ein quantitativer Umsatz an 2H-Heptafluorpropan erreichen. Größere Überschüsse an Chlor sind zwar möglich, jedoch nicht zweckmäßig, da bei der Benutzung eines wirksamen Kondensators die Gefahr besteht, daß unumgesetztes, am Kondensator verflüssigtes, in den Bestrahlungsreaktor zurückfließendes Chlor die Temperatur im Reaktor senkt und somit zu einer Verringerung der Reaktionsgeschwindigkeit führt. Außerdem nimmt durch den Chlorüberschuß der Aufwand bei der destillativen Aufarbeitung zu. Ein größerer Überschuß an Chlor wird aus den gasförmigen Reaktionsprodukten mit Vorteil durch fraktionierte Destillation entfernt. Dabei kann das zurückgewonnene Chlor wieder verwendet werden. Ein Unterschuß an Chlor ist zwar möglich, führt aber zu einer Verringerung des Umsatzes und eine Erhöhung des Aufwandes bei der Aufarbeitung.

Im allgemeinen arbeitet man ohne Zusatz eines Inertgases. Eine Verdünnung durch ein Inertgas, wie z.B. Stickstoff, ist zwar möglich, bringt jedoch keine wesentlichen Vorteile.

Die Bestrahlungsreaktion kann in einem weiten Temperaturbereich von -30° bis +500°C durchgeführt werden; allerdings verläuft die Umsetzung im Bereich von -30° bis + 40°C nur langsam ab. Daher sind Temperaturen über 40°C bevorzugt. Temperaturen im Bereich von 350 bis 500°C sind zwar möglich, aber vom Energieaufwand her nachteilig.

Die Verweilzeit der Ausgangsprodukte und der Endprodukte im Reaktor ist nicht kritisch; sie kann z.B. zwischen einigen Sekunden und einigen Minuten schwanken, ohne daß es durch Eintreten der Nebenreaktionen oder Folgereaktionen zu einer Beeinträchtigung der Rohprodukt-Zusammensetzung kommt. Andererseits ist die Verweilzeit nach oben durch wirtschaftliche Erwägungen begrenzt. Für eine hohe Raum-Zeit-Ausbeute ist es deshalb zweckmäßig, die entstehenden Reaktionsprodukte möglichst umgehend aus dem Reaktor zu entfernen.

Der entstehende Chlorwasserstoff wird im allgemeinen über Kopf durch einen mit festem Kohlendioxid gekühlten Kondensor abgeführt. Das entstehende 2-Chlor-heptafluorpropan, das sich am Boden des Reaktors sammelt, läßt sich ebenfalls kontinuierlich durch einen Hahn abziehen.

Das erfindungsgemäße Verfahren wird im allgemeinen bei Normaldruck ausgeführt; jedoch ist auch die Anwendung von Unterdruck oder Überdruck (z.B. bis 10 bar, vorzugsweise bis 3 bar) in weiten Grenzen möglich. Zur Erzielung hoher Raum-Zeit-Ausbeuten ist die Anwendung von Überdruck bevorzugt.

Bei Umsetzungen im technischen Maßstab ist eine kontinuierliche und gleichmäßige Fahrweise anzustreben. Dem erfindungsgemäßen Verfahren ist ein kontinuierliches Einleiten von Chlor und 2H-Heptafluorpropan, eine kontinuierliche Reaktionsführung, ein kontinuierliches Ausschleusen der Reaktionsprodukte sowie eine kontinuierliche Aufarbeitung ohne weiteres durchführbar. Besondere Vorteile der kontinuierlichen Fahrweise liegen auch in der verbesserten Ausnutzung der Einsatzstoffe sowie in dem geringen Anfall an Abwasser und Abgas.

Der Umsatz an 2H-Heptafluorpropan liegt beim erfindungsgemäßen Verfahren im allgemeinen über 90 %, oft bei über 99 %.

Aufgrund der hohen Selektivität des erfindungsgemäßen Verfahrens liegen die Ausbeuten an 2-Chlor-heptafluorpropan ebenfalls über 90 %, oft über 98 % der Theorie. Das entstandene 2-Chlor-heptafluorpropan fällt in hoher Reinheit an. Die Aufarbeitung ist daher außerordentlich einfach.

Im Hinblick auf Literaturangaben über die fehlende Reaktivität von Wasserstoffatomen in unmittelbarer Nachbarschaft von Trifluormethyl-Gruppen gegenüber Chlor ist es überraschend, daß die Substitution von Wasserstoff durch Chlor in 2H-Hexafluorpropan beim erfindungsgemäßen Verfahren so glatt verläuft.

Bekanntlich wird in vergleichbaren Verbindungen wie

$CF_3-CH_2-CF_3$ und 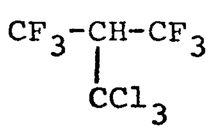

Wasserstoff nicht durch Chlor substituiert (Houben-Weyl, Methoden der organischen Chemie, 1962, Band V/3, S. 594-498).

Das Verfahren wird durch die folgenden Beispiele erläutert.

Beispiel 1

Die Versuchsanordnung besteht aus einem 2 l fassenden Mehrhals-Bestrahlungskolben aus ®DURAN-Glas, in den eine mit einem dünnwandigen Quarzglasrohr versehene UV-Hochdruck-Quecksilber-Tauchlampe eingeführt ist. Außerdem ist der Bestrahlungskolben mit einem Gaseinleitungsrohr, das nahe der Tauchlampe endet, einem Innenthermometer, das in die Nähe der Tauchlampe reicht, einem mit festem $CO_2$ gefüllten Kondensor, der auf eine der oben angebrachten Schliffverbindungen aufgesetzt ist, sowie einem am Boden des Bestrahlungskolbens befindlichen Ablaßhahn ausgestattet. Mit elektrischen Heizstrahlern können die Außenwände in Höhe der Tauchlampe sowie in Höhe des Ablaßhahns von außen zusätzlich geheizt werden. Sowohl der obere Abgang des Kondensors als auch der Ausgang des Ablaßhahns führen in ein Waschgefäß mit Wasser zur Aufnahme des entstehenden Chlorwasserstoffs. Es folgen Waschgefäße, gefüllt mit wässriger 10%iger $Na_2S_2O_3$-Lösung zur Aufnahme des überschüssigen Chlors bzw. gefüllt mit wässriger 10%iger NaOH zur Endwaschung. Es schließen sich zwei Trockentürme an, einer gefüllt mit $CaCl_2$ zur Vortrocknung, der andere gefüllt mit $P_2O_5$ zur Endtrocknung. Das gasförmige Rohprodukt wird schließlich in einer mit $CO_2$ gekühlten Intensivkühlfalle kondensiert.

Elementares Chlor wird einer handelsüblichen Stahlflasche entnommen, mit konzentrierter $H_2SO_4$ getrocknet, mit einem Strömungsmesser dosiert und mit dem $CF_3CHFCF_3$-Gasstrom gemischt.

2H-Heptafluorpropan wird gemäß DE-OS 2 712 732 durch Hydrofluorierung von Hexafluorpropen in Gegenwart eines Chromoxifluoridkatalysators hergestellt. Es wird mit $P_2O_5$ getrocknet, mit einem Strömungsmesser dosiert und mit dem $Cl_2$-Strom gemischt.

Vor Beginn der Photochlorierung wird der zuvor mit $N_2$ gespülte Reaktionsraum durch die eingeschaltete UV-Lampe vorgeheizt; dabei werden im Reaktorraum Temperaturen von ca. 150° bis 180°C erreicht. In unmittelbarer Nähe der Tauchlampe werden auf dem Quarzglas Temperaturen von 260° bis 300°C gemessen.

Zur Photochlorierung von 2H-Heptafluorpropan werden bei Temperaturen von 120° bis 90°C innerhalb von 2 h insgesamt 76 g (0,45 Mol) $CF_3CHFCF_3$ und 33 g (0,46 Mol) Chlor, entsprechend einem Molverhältnis von $CF_3CHFCF_3$ : $Cl_2$ wie 1 : 1,02 eingeleitet. Während des Versuchs wird der sich entwickelnde Chlorwasserstoff, der vom Kondensor nicht zurückgehalten wird, in der Waschwasservorlage absorbiert. Nach Beendigung der Umsetzung wird mit $N_2$ nachgespült, um den gesamten Chlorwasserstoff im Waschwasser herauszuwaschen. In diesem Beispiel (1) werden die übrigen gasförmigen Reaktionsprodukte, die sich am Boden des Bestrahlungsgefäßes sammeln, erst nach Beendigung der Umsetzung durch den am Boden befindlichen Ablaßhahn abgeführt.

Im Waschwasser werden 0,44 Mol HCl titriert; HF wird nicht festgestellt. Das kondensierte Rohprodukt wird gaschromatographisch an einer ®PORAPAK-Säule untersucht. Dabei findet man:

| | |
|---|---|
| $CF_3CClFCF_3$ | 98,1 % |
| $CF_3CHFCF_3$ | 1,7 % |
| $CF_3CClFCClF_2$ | 0,1 % |
| $CF_3CF_3$ | $<$ 0,05 % |
| $CF_3CF_2CF_3$ | $<$ 0,05 % |

Die beiden letztgenannten Komponenten stellen nicht umgesetzte Verunreinigungen des eingesetzten Heptafluorpropans dar; $CF_3CClFCClF_2$ entsteht durch Chlorierung von $C_3F_6$, das ebenfalls im Ausgangsprodukt in Spuren enthalten.

Das flüssige Rohprodukt wiegt 88 g. Damit beträgt die Ausbeute an $CF_3CClFCF_3$ 95,4 % d.Th. bezogen auf umgesetztes $CF_3CHFCF_3$. Eine weitere Identifizierung von 2-Chlor-1,1,1,2,3,3,3-heptafluorpropan erfolgt durch $^{19}F$-NMR-Messung, Infrarot-Aufnahme und Bestimmung des Siedepunktes, der bei -2°C liegt.

Beispiel 2

In der Apparatur von Beispiel (1) werden zur Photochlorierung von $CF_3CHFCF_3$ bei Temperaturen von 115° bis 110°C zu Beginn des Versuchs und Temperaturen von 35° bis 20°C bei Ende des Versuchs innerhalb von 5,5 h insgesamt 467 g (2,75 Mol) $CF_3CHFCF_3$ und 198 g (2,79 Mol) $Cl_2$, entsprechend einem Molverhältnis von $CF_3CHFCF_3$ : $Cl_2$ wie 1 : 1,01, eingeleitet. Die sich am Boden des Bestrahlungskolbens sammelnden kondensierten Reaktionsprodukte werden wie im Beispiel (1) erst nach Beendigung des Versuchs über den Ablaßhahn abgetrennt und gewaschen.

Im Waschwasser der Gase werden 1,84 Mol HCl titriert. Die gaschromatographische Analyse ergibt folgende Zusammensetzung für das kondensierte Rohprodukt:

| | |
|---|---|
| $CF_3CClFCF_3$ | 67,5 % |
| $CF_3CHFCF_3$ | 32,1 % |
| 3 andere Komponenten je | 0,1 % |

Das Kondensat wiegt 512 g. Die Ausbeute an $CF_3CClFCF_3$ beträgt damit 90,5 % d.Th. bezogen auf umgesetztes $CF_3CHFCF_3$; der Umsatz an $CF_3CHFCF_3$ erreicht jedoch nur ca. 68 %.

Beispiel 3

In der Apparatur von Beispiel (1) werden bei Temperaturen von 120° bis 110°C, die während des gesamten Versuchs eingehalten werden können, innerhalb von 5 h insgesamt 495 g (2,91 Mol) $CF_3CHFCF_3$ und 217 g (3,06 Mol)

Chlor, entsprechend einem Molverhältnis von $CF_3CHFCF_3$ : $Cl_2$ wie 1 : 1,05, eingegast. Über den am Boden des Bestrahlungskolbens befindlichen Ablaßhahn werden die sich sammelnden Reaktionsprodukte s t ä n d i g und gleichmäßig bis auf eine stets verbleibende Restmenge von ca. 10 bis 15 ml ausgeschleust und dann gewaschen.

Im Waschwasser der Gase werden 2,76 Mol HCl gefunden. Die gaschromatographische Messung für das aufgefangene kondensierte Rohprodukt ergibt folgende Zusammensetzung:

| | |
|---|---|
| $CF_3CClFCF_3$ | 95,3 % |
| $CF_3CHFCF_3$ | 3,9 % |
| Rest Nebenkomponenten | 0,8 %. |

Das Kondensat wiegt 585 g. Damit beträgt die Ausbeute an $CF_3CClFCF_3$ bei 97,6 % d.Th. bezogen auf umgesetztes $CF_3CHFCF_3$.

<u>Beispiel 4</u>

In der Apparatur von Beispiel (1) werden bei Temperaturen von 135° bis 125°C innerhalb von 7,5 h insgesamt 1080 g (6,35 Mol) $CF_3CHFCF_3$ und 526 g (7,41 Mol) Chlor, entsprechend einem Molverhältnis wie $CF_3CHFCF_3$ : $Cl_2$ wie 1 : 1,17 eingeleitet. Durch elektrische Heizstrahler werden die Außenwände des Kolbens in Höhe der Tauchlampe und des Ablaßhahns zusätzlich geheizt. Wie in Beispiel (3) werden die sich sammelnden Reaktionsprodukte ständig und gleichmäßig aus dem Kolben ausgeschleust.

Im Waschwasser werden 6,31 Mol HCl gefunden. Die gaschromatographische Analyse ergibt für das Rohprodukt folgende Zusammensetzung:

| | |
|---|---|
| $CF_3CClFCF_3$ | 99,7 % |
| $CF_3CHFCF_3$ | 0,15 % |
| $CF_3CClFCClF_2$ | $<$0,02 % |

$CF_3CF_2CF_3$ $< 0,05$ %
$CF_3CHFCClF_2$ $0,1$ %

Das Kondensat hat ein Gewicht von 1289 g; damit liegt die Ausbeute an $CF_3CClFCF_3$ bei 99,1 % d.Th. bezogen auf umgesetztes $CF_3CHFCF_3$. Der Umsatz an $CF_3CHFCF_3$ beträgt 99,85 % d.Th.

PATENTANSPRÜCHE

1. Verfahren zur selektiven Herstellung von 2-Chlor-1,1,1,2,3,3,3-heptafluorpropan (I), dadurch gekennzeichnet, daß man 1,1,1,2,3,3,3-Heptafluorpropan (II) in der Gasphase mit elementarem Chlor in Gegenwart von energiereicher Strahlung bei Reaktionstemperaturen im Bereich von -30° bis + 500°C umsetzt.

2. Verfahren nach Anspruch 1, dadurch gekennzeichnet, daß man II mit Chlor im Molverhältnis von 1 : 1,0 bis 1,5, bevorzugt von 1 : 1,05 bis 1,15 Mol umsetzt.

3. Verfahren nach Anspruch 2, dadurch gekennzeichnet, daß man bei Reaktionstemperaturen im Bereich von 40° bis 450°C arbeitet.

Europäisches Patentamt

# EUROPÄISCHER RECHERCHENBERICHT

0039471

Nummer der Anmeldung

EP 81 10 3200.2

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | US - A - 2 658 928 (J.H. SIMONS et al.)<br>* Beispiel 2 *<br>-- | 1 | C 07 C 19/08<br>C 07 C 17/10 |
| D,A | GB - A - 839 034 (W.T. MILLER)<br>* Seite 4, Zeilen 58 bis 65 *<br>-- | 1 | |
| A | Chemical Abstracts Band 49, Nr. 3, 1955<br>Columbus, Ohio, USA<br>R.N. HASZELDINE "Reactions of fluorocarbon radicals. XII. The synthesis of fluorocarbons and of fully fluorinated iodo-, bromo-, and chloroalkanes"<br>Spalte 1533bc<br>& Journal of the Chemical Society, Band 1953, Seiten 3761 bis 3768<br>-- | 1 | RECHERCHIERTE SACHGEBIETE (Int. Cl.³)<br>C 07 C 17/10<br>C 07 C 19/08 |
| A | Chemical Abstracts Band 57, Nr. 5, 1962<br>Columbus,Ohio, USA<br>R.D. CHAMBERS et al. " Organometallic and metalloid compounds made from heptafluoro-2-iodopropane, and their properties"<br>Spalten 5938f und 5939b<br>& Journal of the Chemical Society, Band 1962, Seiten 1993 bis 1999<br>---- | 1 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 24-07-1981 | KNAACK |